# EUROPÄISCHE PATENTSCHRIFT

(11) **EP 3 735 919 B1**
(45) Veröffentlichungstag und Bekanntmachung des Hinweises auf die Patenterteilung: **21.06.2023**
(21) Anmeldenummer: 19173031.6
(22) Anmeldetag: 07.05.2019
(51) Int. Cl.: A61B 17/29, A61B 17/16, A61B 90/00

(54) **MEDIZINISCHES INSTRUMENT**
MEDICAL INSTRUMENT
INSTRUMENT MÉDICAL

(43) Veröffentlichungstag der Anmeldung: 11.11.2020
(73) Patentinhaber: Richard Wolf GmbH, 75438 Knittlingen (DE)
(72) Erfinder: Prestel, Stephan, 76287 Rheinstetten (DE)
(74) Vertreter: Patentanwälte Vollmann Hemmer Lindfeld Partnerschaft mbB

(56) Entgegenhaltungen:
- EP-A1- 2 702 951
- WO-A2-2005/079679
- DE-A1-102010 013 296
- DE-U1-202008 001 675
- DE-U1-202009 002 433

## Beschreibung

Die Erfindung betrifft ein medizinisches Instrument, insbesondere ein endoskopisches Instrument mit den im Oberbegriff des Anspruchs 1 angegebenen Merkmalen. Insbesondere betrifft die Erfindung ein Zangenmaulteilinstrument, beispielsweise in Form einer Stanze, Zange, einer Schere, einer Klemme oder dergleichen.

Bei derartigen Instrumenten, insbesondere wenn sie für chirurgische Eingriffe ausgelegt sind, ist es in der Regel erforderlich, zum Zwecke der Reinigung entweder das Instrument in seine Einzelteile zu zerlegen oder aber, was wesentlicher einfacher und praktikabler ist, es in eine Reinigungsposition zu verbringen, in welcher das Instrument gründlich gereinigt werden kann, typischerweise in einer medizinischen Spülmaschine, einem Ultraschallbad oder dergleichen. Dieses Problem wird bei endoskopischen Instrumenten noch dadurch verstärkt, dass eine Zug-/Druckstange innerhalb eines langgestreckten Schaftes geführt ist, dort sind Zwischenräume gebildet, die in der Arbeitsposition nicht oder nur sehr schwer erreichbar sind.

Ein typisches Instrument dieser Art ist aus DE 9421125 U1 bekannt. Die dort beschriebene chirurgische Zange hat ein proximales Bedienteil mit einem schwenkbaren und einem feststehenden Griffteil, welches über einen langgestreckten nach oben offenen Schaft mit dem distalen Zangenmaulteil verbunden ist, dessen schwenkbewegliches Maulteil mittels einer Zug-/Druckstange bewegt wird, die sich in Arbeitsposition innerhalb des Schaftes befindet und die zu Reinigungszwecken aus der nutartigen Ausnehmung des Schaftes nach oben ausschwenkbar und bei Erreichen einer 90°-Stellung zum Schaft auch entfernbar ist.

Die Entfernbarkeit ist eher nachteilig, da nicht sichergestellt ist, dass das Instrument in seiner Gänze beim Reinigungsvorgang zusammen bleibt und so die Möglichkeit besteht, das ein Instrumententeil verlorengeht oder mit dem eines anderen Instrumentes vertauscht wird, was problematisch sein kann. Ein weitere Nachteil besteht darin, dass in der leicht aufgeschwenkten Stellung zwar sowohl der Schaft als auch die Zug-/Druckstange gut zugänglich und somit z.B. in einer medizinischen Spülmaschine gereinigt werden können, dass jedoch stets eine Hilfsvorrichtung erforderlich ist, welche sicherstellt, dass die Instrumententeile während der Reinigung in dieser Reinigungsposition verbleiben und nicht die Zug-/Druckstange wieder in den Schaft zurückgeführt wird.

Bei dem aus DE 102012006080 A1 bekannten Instrument ist zwar sichergestellt, dass dieses nicht versehentlich zum Zwecke der Reinigung in seine Einzelteile zerlegt wird, das Problem, dass während der Reinigung die Reinigungsposition durch eine Hilfsvorrichtung fixiert werden muss, ergibt sich auch dort.

Ähnlich verhält es sich bei dem aus DE 20 2009 002 433 U1 bekannten Instrument, das in seiner Reinigungsposition nur noch eine Anlenkstelle zwischen der sich gegenüber der gesamten Länge des Instrumentes erstreckenden Zug-/Druckstange und der zugehörigeren Führung aufweist. Die gleiche Problematik ergibt sich auch bei dem aus DE 20 2008 001 675 U1, WO 2005/079679 A2 und EP 2 702 951 A1 bekannten Instrumenten mit Zangenmaulteilen.

Insoweit günstiger ist das aus DE 10 2010 013 296 A1 bekannte Instrument, bei welchem die Zug-/Druckstange in der Reinigungsposition an beiden Enden festgelegt ist.

Ausgehend von dem letztgenannten Stand der Technik liegt der Erfindung die Aufgabe zugrunde, ein gattungsgemäßes medizinisches Instrument zu verbessern, insbesondere so auszubilden, dass eine zuverlässige Reinigung auf einfache Weise gewährleistet ist.

Diese Aufgabe wird gemäß der Erfindung durch ein medizinisches Instrument mit den in Anspruch 1 angegebenen Merkmalen gelöst. Vorteilhafte Ausgestaltungen der Erfindung sind in den Unteransprüchen, der nachfolgenden Beschreibung und den Zeichnungen angegeben.

Das medizinische Instrument, bei dem es sich insbesondere um ein endoskopisches Instrument handelt, weist ein distales Zangenmaulteil, typischerweise eine Stanze, Zange, eine Schere, eine Klemme oder dergleichen auf, welches über ein proximales Bedienteil betätigt werden kann. Zwischen Bedienteil und Maulteil ist ein dies verbindendes einseitig offenes Führungsteil vorgesehen, in welchem eine Zug-/Druckstange axial verschiebbar geführt ist. Diese Zug-/Druckstange ist mit ihrem distalen Ende an einem bewegbaren Teil des Maulteils oder einem damit verbundenen Bauteil angelenkt und mit ihrem proximalen Ende in einer Arbeitsposition mit einem beweglich am Bedienteil angeordneten Griffteil bewegungsgekoppelt. Dabei kann die Zug-/Druckstange aus ihrer Arbeitsposition in eine Reinigungsposition verbracht, insbesondere geschwenkt werden, in welcher zumindest ein Stangenabschnitt oder vorteilhaft die gesamte Stange außerhalb des Führungsteils angeordnet und das proximale Stangenende beabstandet zum Bedienteil ist. Gemäß der Erfindung ist am Instrument selbst eine Spreizvorrichtung vorgesehen, welche die Zug-/Druckstange in ihrer Reinigungsposition hält. Dabei ist die Spreizvorrichtung gemäß der Erfindung so ausgebildet, dass sie die Zug-/Druckstange nicht nur in ihrer Reinigungsposition hält, sondern zum selbsttätigen Überführen und Halten der Zug-/Druckstange in die beziehungsweise in der Reinigungsposition ausgebildet. Gemäß der Erfindung weist die Spreizvorrichtung eine Feder auf, die vorzugsweise vorgespannt ausgebildet ist und welche die Zug-/Druckstange in Richtung von der Arbeitsposition zur Reinigungsposition hin kraftbeaufschlagt. Diese Feder, die auch aus mehreren in Reihe oder parallel geschalteten Federelementen aufgebaut sein kann, sorgt also einerseits für die Bewegung der Zug-/Druckstange von der Arbeitsposition in die Reinigungsposition und andererseits dafür, dass die Zug-/Druckstange in der Reinigungsposition verbleibt. Dabei ist insbesondere die Vorspannung hilfreich, um eine ausreichende Haltekraft auch in der Reinigungsposition zu gewährleisten. Die Rückführung erfolgt typischerweise manuell entgegen dieser Federkraft

Grundgedanke der vorliegenden Erfindung ist es somit, das Instrument so auszustatten, dass es in beiden Positionen, nämlich in seiner Arbeitsposition aber auch in seiner Reinigungsposition zuverlässig gehalten wird. Hierfür ist gemäß der Erfindung eine Spreizvorrichtung vorgesehen, welche die Zug-/Druckstange in ihrer Reinigungsposition hält bzw. fixiert. Eine solche Spreizvorrichtung kann beispielsweise durch einen ausklappbaren Hebel gebildet sein, welcher die Zug-/Druckstange in der ausgeschwenkten, d.h. in der Reinigungsposition gegen ein Rückschwenken sichert. Hierbei kann beispielsweise der vorgenannte Schwenkhebel federkraftbeaufschlagt sein, so dass nach Lösen einer Sicherung die Zug-/Druckstange selbsttätig in die Reinigungsposition überführt wird und in dieser Position durch die Spreizvorrichtung gehalten ist. Die Spreizvorrichtung kann durch eine teleskopierbare Stange oder einen teleskopierbaren Bolzen gebildet sein, welcher federkraftbeaufschlagt ist, so dass auf die Zug-/Druckstange eine Kraft in Richtung ihrer Reinigungsposition ausgeübt wird, so dass nach Lösen einer Verriegelung oder einer anderen Sicherung die Zug-/Druckstange durch die Federkraft von ihrer Arbeitsposition in die Reinigungsposition verfahren und durch die Federkraft dort gehalten werden kann.

Der Führungsteil, welcher zwischen Bedienteil und Maulteil angeordnet ist, kann beispielsweise so ausgebildet sein, dass aus Zug-/Druckstange einerseits und einem sich längs erstreckenden Teil des Führungsteils andererseits ein kreisrunder Querschnitt gebildet ist, wobei in einer Teilungsebene die Bauteile flächig aufeinander gleiten. Zur Führung können dann ein oder mehrere die Zug-/Druckstange zumindest abschnittsweise teilumfassende Vorsprünge vorgesehen sein. Eine solche Anordnung wird bei vergleichsweise kurzen Instrumenten praktikabel sein. Bei endoskopischen Instrumenten hingegen ist es vorteilhaft, den einseitig offenen Führungsteil durch ein Schaftbauteil zu bilden, insbesondere durch einen einseitig zumindest abschnittsweise offenen Schaft, wobei die Öffnung zweckmäßigerweise zu der von den Griffteilen abgewandten Seite des Schaftes nutartig angeordnet ist und sich über die gesamte Länge oder zumindest bis nahe zum distalen Schaftende erstreckt.

Dieser einseitig offene Schaft bildet dabei nicht nur die mechanische Verbindung zwischen Bedienteil und Maulteil sondern kann darüber hinaus auch noch einen Saug-Spül-Kanal oder andere bzw. weitere Bauteile umfassen.

Um zu verhindern, dass die Reinigungsposition der Zug-/Druckstange unbeabsichtigt eingelegt wird, ist es vorteilhaft eine Verriegelung für die Zug-/Druckstange und/oder für die Spreizvorrichtung vorzusehen, welche das Instrument in seiner Arbeitsposition festlegt. Diese Verriegelung sollte zumindest rastend sein um sicherzustellen, dass sie nicht versehentlich gelöst wird.

Eine solche Verriegelung ist vorteilhaft durch einen Ring gebildet, welcher das einseitig offene Führungsteil zumindest abschnittsweise umgibt und welcher drehbar am Führungsteil und/oder am Bedienteil vorzugsweise rastend festgelegt ist. Dieser Ring weist eine Durchgangsnut auf, welche in einer Drehstellung, in der die Durchgangsnut mit der offenen Seite der Führung fluchtet, einen Freiraum zum Hindurchführen der Zug-/Druckstange bildet, so dass in dieser Stellung die Zug-/Druckstange durch die Federkraft oder ggf. auch durch manuelle Kraft durch die Durchgangsnut hindurch aus der Führung, insbesondere dem nach oben offenen Schaft aus dem Schaft hinaus und in die Reinigungsposition hineinbewegt wird. Da die Zug-/Druckstange an einem bewegten Teil des Maulteils angelenkt ist, wird dieses Gelenk vorteilhaft zugleich als Schwenkachse für die Zug-/Druckstange genutzt, wenn diese aus der Arbeitsposition in die Reinigungsposition geschwenkt wird. Wenn der Ring im Bereich des proximalen Endes der Führung bzw. des Schaftes angeordnet ist, also etwa dort wo der Schaft in das Bedienteil mündet, dann kann dieser Ring vergleichsweise schmal ausgebildet sein, da er nur geringe Kräfte aufzunehmen hat und gleichzeitig vom Bedienteil her günstig zu erreichen ist.

Das Bedienteil weist vorteilhaft einen Grundkörper auf, der fest mit der Führung bzw. dem Schaft verbunden ist und einen schwenkbar daran angeordneten Griffteil aufweist. Dabei ist der Griffteil im distalen Bereich des Bedienteils angeordnet und weist vorteilhaft einen Bolzen auf, der axial beweglich innerhalb des Griffteils angeordnet ist und dessen Achsrichtung im Wesentlichen quer zur Längsachse der Zug-/Druckstange angeordnet ist. Dieser Bolzen ist mit seinem einen Ende an der Zug-/Druckstange angelenkt und mit dem anderen Ende in einer Ausnehmung des Griffteils axial beweglich geführt, so dass im Instrument kein zusätzlicher Raumbedarf für diesen Bolzen erforderlich ist.

Gemäß einer vorteilhaften Ausgestaltung der Erfindung ist der Bolzen federkraftbeaufschlagt, und zwar durch eine Schraubenfeder, welche in der Ausnehmung des Griffteils angeordnet ist und die sich einerseits am Ende der Ausnehmung im Griffteil und andererseits am Bolzen abstützt. Diese Ausnehmung kann bei Verwendung eines im Querschnitt kreisrunden Bolzens als Sacklochbohrung ausgebildet sein, dann stützt die Schraubenfeder am Boden dieser Bohrung ab und an der Stirnseite des Bolzens oder aber umgreift den in der Ausnehmung geführten Teil des Bolzens und stützt sich an einer Abstufung dieses Bolzens ab. Statt einer Sacklochbohrung kann die Ausnehmung auch an geeigneter Stelle durch einen Querstift begrenzt sein.

Vorteilhaft ist ein Anschlag vorgesehen, welcher die Ausfahrbewegung des Bolzens aus der Ausnehmung begrenzt und somit die Schwenkbewegung der Zug-/Druckstange in die Reinigungsposition definiert.

Um sicherzustellen, dass beim Verfahren von der Arbeitsposition in die Reinigungsposition auch der Griffteil eine definierte Position einnimmt, sind vorteilhaft zwischen Bolzen und Grundkörper des Bedienteils Formschlussmittel vorgesehen, welche ein Ausfahren des Bolzens nur bei einer vorgegebenen Stellung des Griffteils erlauben und welche die Schwenkbarkeit des Griffteils in ausgefahrener Stellung des Bolzens blockieren. Diese definierte Stellung ist vorzugsweise aber nicht notwendigerweise die geöffnete Stellung des Griffteils.

Die vorstehenden Ausführungen betreffen insbesondere eine Konstruktion, bei welcher der schwenkbar angeordnete Griffteil im Wesentlichen distalseitig des Grundkörpers angeordnet ist und am Grundkörper ein Gegengriffteil vorgesehen ist. Es versteht sich jedoch, dass bei kinematisch umgekehrt ausgebildetem Bedienteil die Anordnung in analoger Weise erfolgen kann. Dann ist der schwenkbare Griffteil im Wesentlichen im proximalseitigen Bereich des Grundkörpers angeordnet.

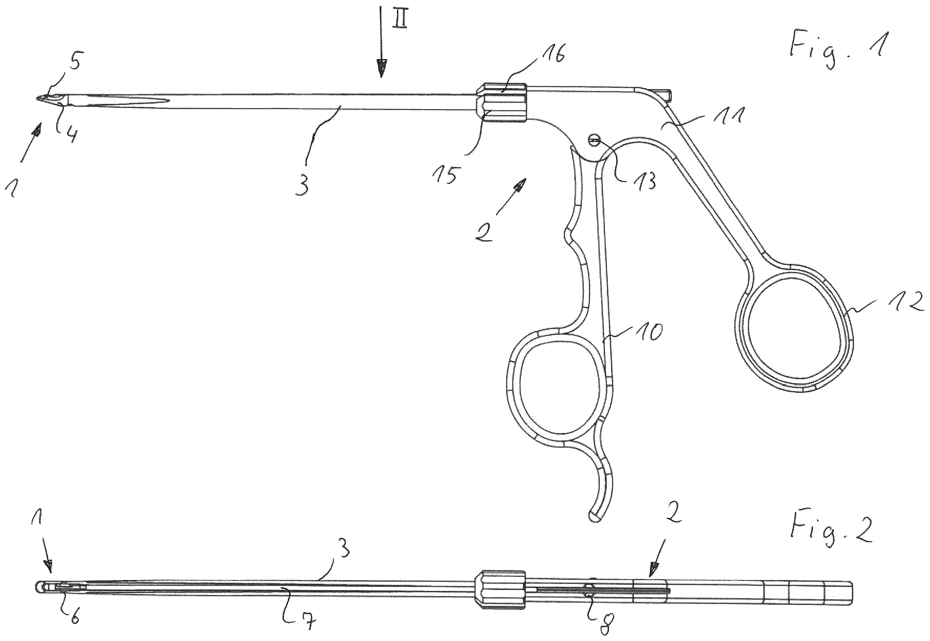

Die Erfindung ist nachfolgend anhand eines in den Zeichnungen dargestellten Ausführungsbeispiels näher erläutert. Es zeigen
- Figur 1: in stark vereinfachter schematischer Darstellung eine Seitenansicht auf eine medizinische Zange,
- Figur 2: eine Ansicht in Richtung des Pfeils II in Figur 1,

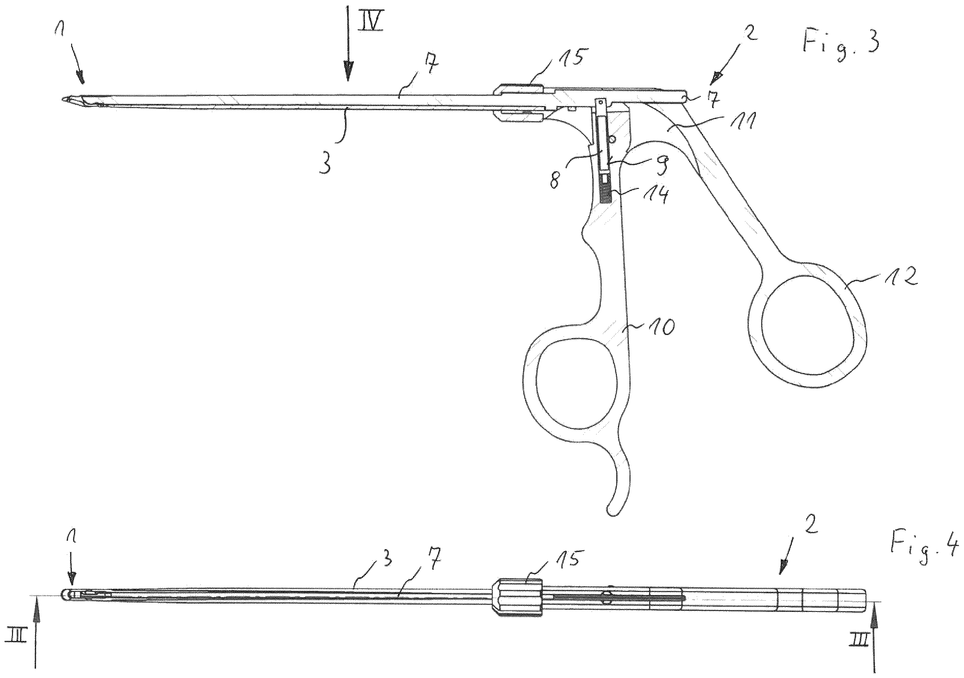

- Figur 3: die Zange gemäß Figur 1 in teilgeschnittener Darstellung entsprechend der Schnittlinie III-III in Figur 4,
- Figur 4: eine Draufsicht in Richtung des Pfeils IV in Figur 3,
- Figur 5: eine Darstellung des Instruments entsprechend Figur 3 jedoch in Reinigungsposition,
- Figur 6: eine Draufsicht auf das Instrument gemäß Figur 5 in Richtung des Pfeils VI,

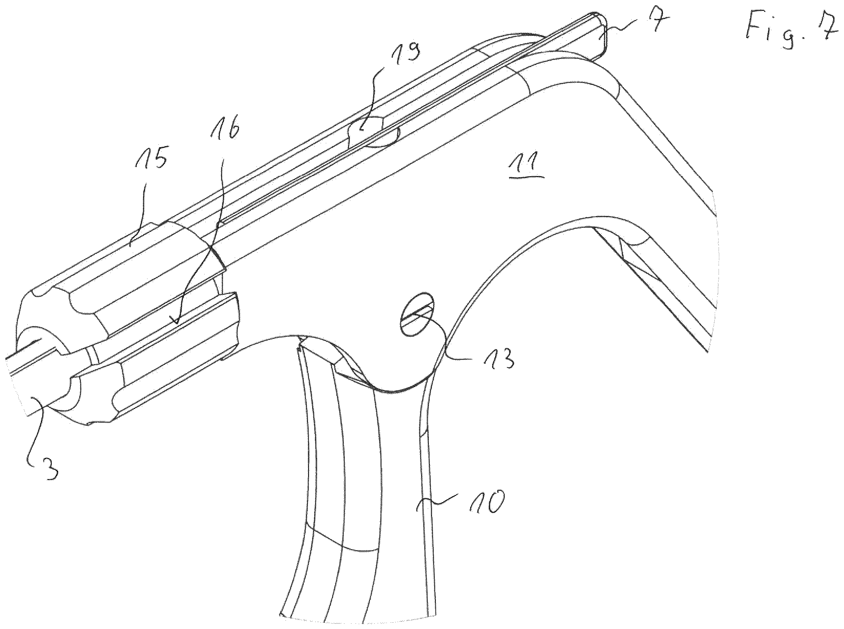

- Figur 7: in perspektivischer vergrößerter Darstellung einen bedienteilseitigen Teil des Instrumentes in Arbeitsposition,
- Figur 8: den Teil gemäß Figur 7 in Reinigungsposition und

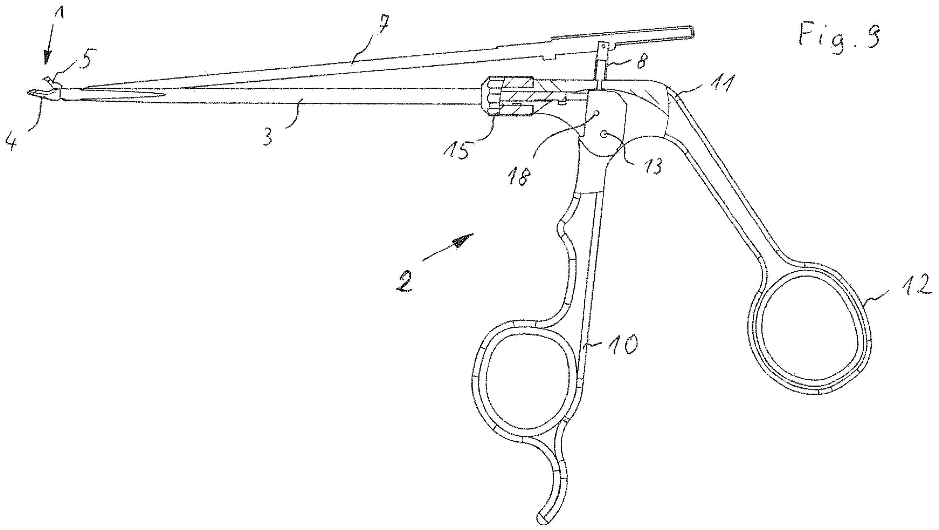

- Figur 9: eine Darstellung wie Figur 1 mit Teilschnitt im Griffbereich
Bei dem in den Figuren dargestellten Instrument handelt es sich um eine endoskopische Zange. Diese weist ein distales Maulteil 1 sowie ein proximales Bedienteil 2 auf, die durch ein Führungsteil in Form eines einseitig offenen Schaftes 3 mechanisch miteinander verbunden sind.

Das Maulteil 1 weist in den Figuren ein unteres feststehendes Teil 4 auf, welches mit einem schwenkbeweglichen Teil 5 das Zangenmaul bildet. Das schwenkbewegliche Teil 5 ist über eine im distalen Bereich des Schaftes 3 festgelegte Schwenkachse 6 gegenüber dem starren Teil 4, welches Teil des Schaftes 3 bildet, schwenkbar gelagert. Proximalseitig ist an das schwenkbewegliche Teil 5 das distale Ende einer Zug-/Druckstange 7 angelenkt, welche in ihrer Arbeitsposition innerhalb des Schaftes 3 axial bewegbar, quer dazu jedoch durch diesen geführt ist. Die Zug-/Druckstange 7 ist nahe ihrem proximalen Ende an das freie Ende eines Bolzens 8 angelenkt, welcher in einer Ausnehmung in Form einer Sacklochbohrung 9 in einem schwenkbeweglichen Griffteil 10 axial begrenzt bewegbar geführt ist. Der schwenkbewegliche Griffteil 10 bildet den beweglichen Teil des Bedienteils 2, dessen feststehender Teil durch einen Grundkörper 11 gebildet ist, an dessen proximalen Ende ein Gegengriffteil 12 starr angeordnet ist. Der schwenkbewegliche Griffteil 10 ist über eine Achse 13 bewegbar am Grundkörper 11 gelagert, die Achse 13 ist unmittelbar neben der Bohrung 9 quer zu dieser angeordnet. Der Bolzen 8 ist durch eine vorgespannte Schraubenfeder 14 kraftbeaufschlagt, die sich mit einem Ende am Boden der Sacklochbohrung 9 und mit ihrem anderen Ende am Bolzen 8 abstützt.

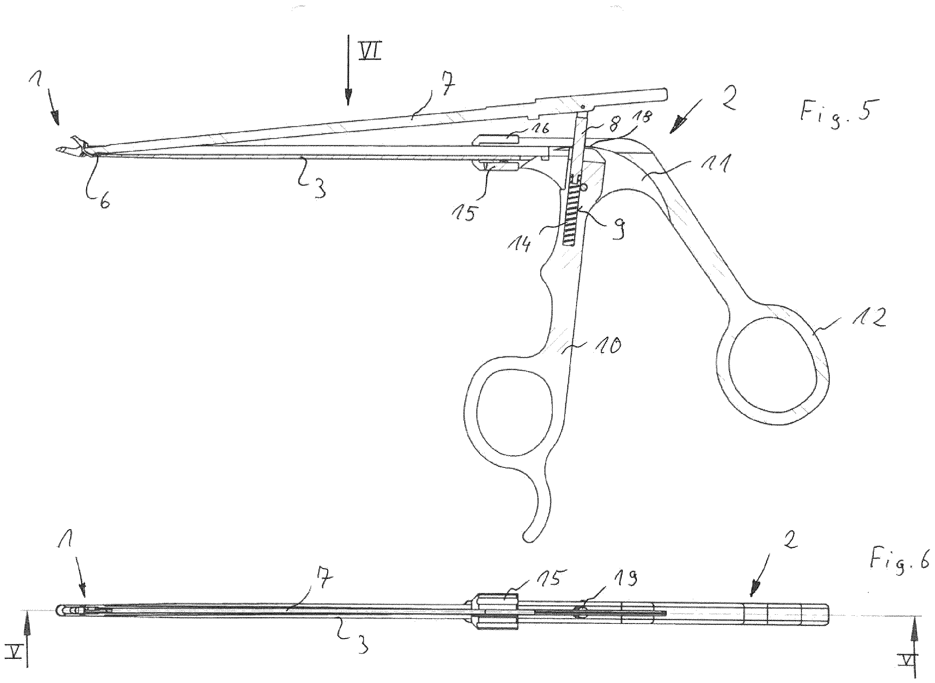

Die Zug-/Druckstange 7 ist in ihrer Arbeitsposition innerhalb des Schaftes 3 geführt, proximalseitig darüber hinaus in einer nach oben offenen Nut im Grundkörper 11 des Bedienteils 2. Die Bewegungskopplung zwischen der Zug-/Druckstange 7 und dem schwenkbeweglichen Griffteil 10 erfolgt über den Bolzen 8, der an der Zug-/Druckstange 8 im proximalen Bereich angelenkt ist. Wird also der schwenkbewegliche Griffteil 10 in der Arbeitsposition aus seiner geöffneten Stellung (Figur 5 allerdings in Reinigungsposition) durch Verschwenken des Griffteils 10 in Richtung zum Gegengriffteil 12 bewegt, so wird die Zug-/Druckstange 7 innerhalb der Führung 3, die durch den nach oben offenen Schaft 3 gebildet ist, axial verschoben, wodurch das schwenkbare Maulteil 5 in Bezug auf das feststehende Teil 4 des Maulteils 1 bewegt wird, d.h. das Zangenmaul geschlossen wird.

Zum Reinigen des Instrumentes ist insbesondere der von außen nicht oder nur schwer zugängliche Teil zwischen der Zug-/Druckstange 7 und dem Schaft 3 freizulegen. Hierzu muss die Zug-/Druckstange 7 um ihre Achse, mit der sie am schwenkbeweglichen Teil 5 des Maulteiles 1 angelenkt ist, verschwenkt werden, nämlich in die in den Figuren 5, 6 und 8 dargestellte Reinigungsposition, in welcher das proximale Ende der Zug-/Druckstange 7 mit Abstand zum Bedienteil 2 angeordnet ist und die Zug-/Druckstange 7 nach oben aus dem Schaft 3 herausgeschwenkt ist.

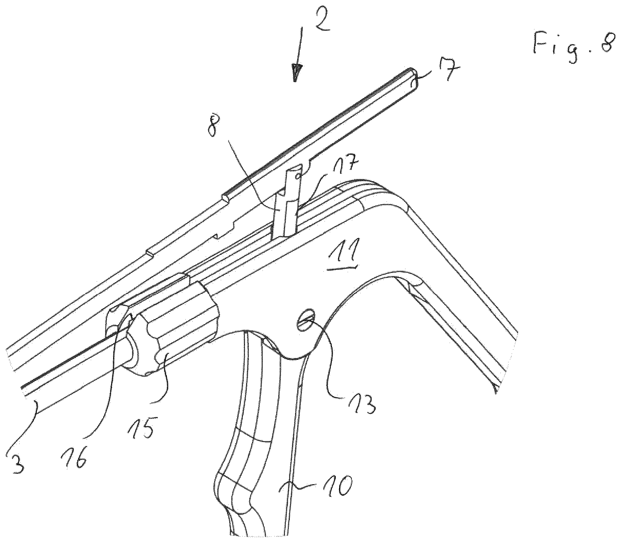

Um zu verhindern, dass diese durch die Schraubenfeder 14 und den Bolzen 8 in Richtung zur Reinigungsposition kraftbeaufschlagte Zug-/Druckstange 7 beim Handhaben des Instrumentes bei der bestimmungsgemäßen Arbeit aus dem Schaft 3 herausschwenkt, ist ein Sicherungsring 15 vorgesehen, welcher drehbar am distalen Abschnitt des Bedienteils 2 gelagert ist und der den proximalen Teil des Schaftes 3 umgibt. Dieser drehbar angeordnete Ring 15 weist eine parallel zum Schaft 3 verlaufende Nut 16 auf, die in der Figur 8 dargestellten Stellung, in welcher die Nut 16 mit der nutartigen Öffnung im Schaft 3 fluchtet, einen Freiraum bildet, durch welchen die Zug-/Druckstange 7 aus der Arbeitsposition in die Reinigungsposition schwenken kann. Dieser Ring 15 hat Raststellungen, so dass je nach Drehstellung verhindert ist, dass dieser versehentlich in eine nicht gewünschte Position gelangt.

Die Kraftübertragung vom schwenkbeweglichen Griffteil 10 auf die Zug-/Druckstange 7 erfolgt mittels des Bolzens 8, der jedoch in einem begrenzten Bereich an zwei Seiten 17 abgeflacht ausgebildet ist, so dass er bis zu einem hierfür vorgesehenen Anschlag 18 im Griffteil 10 begrenzt bewegbar ist, nämlich dann, wenn der Bolzen 8 soweit in die Bohrung 9 eingefahren ist, dass die Zug-/Druckstange 7 innerhalb des Schaftes 3 angeordnet ist. Eine Ausnehmung im Grundkörper 11 für den Bolzen 8 ist dabei so ausgestaltet, dass ein Bewegen des Bolzens 8 in die Reinigungsposition nur dann möglich ist, wenn der schwenkbewegliche Griffteil 10 vollständig geöffnet ist, d.h. in die Stellung verbracht ist, in welcher Griffteil 10 und Gegengriffteil 12 maximal voneinander beabstandet sind und das Maulteil 1 vollständig geöffnet ist. Es ist also eine Ausnehmung 19 innerhalb des Grundkörpers 11 vorgesehen, in welche der Bolzen 8 bewegt werden kann, allerdings ist diese Ausnehmung 19 so angeordnet, dass sie nur in der geöffneten Stellung mit der Bohrung 9 im schwenkbeweglichen Griffteil 10 fluchtet. Auf diese Weise ist durch einfachen Formschluss sichergestellt, dass beim Schwenken der Zug-/Druckstange 7 von der Arbeitsposition in die Reinigungsposition stets das Maulteil 1 geöffnet und auch Griffteil 10 und Gegengriffteil 12 einen weitestmöglichen Abstand haben. Darüber hinaus ist in dieser Stellung sichergestellt, dass eine Bewegung des schwenkbeweglichen Griffteils 10 um seine Schwenkachse 13 dann durch den Bolzen 8 blockiert ist, eine Fehlbedienung somit ausgeschlossen ist. Gleichzeitig ist innerhalb des Griffteiles 10 für den Bolzen 8 ein Anschlag 18 vorgesehen, welcher die Ausfahrbewegung des Bolzens 8 begrenzt und somit die Schwenkstellung der Zug-/Druckstange 7 begrenzt und damit in der Reinigungsposition festlegt. Dieser Anschlag 18 sorgt dafür, dass nur der Teil des Bolzens 8 durch die Ausnehmung 19 im Grundkörper 11 hindurchgelangt, welcher die abgeflachten Seiten 17 aufweist, nicht dagegen der sich daran anschließende Teil des Bolzens mit kreisrundem Querschnitt. Im vorstehend dargestellten Ausführungsbeispiel ist die Ausnehmung 19 durch eine im Querschnitt kreisrunde Bohrung gebildet. Diese kann jedoch auch langlochförmig ausgebildet sein, um in der Reinigungsposition eine gewisse Beweglichkeit der Teile zueinander zu gewährleisten und so insbesondere die Vorreinigung des Instrumentes zu verbessern. Alternativ kann im Bolzen auch ein Langloch vorgesehen sein, durch den ein Querstift als Anschlag geführt ist.

### Bezugszeichenliste

- 1: Maulteil
- 2: Bedienteil
- 3: Schaft
- 4: feststehendes Teil von 1
- 5: schwenkbewegliches Teil von 1
- 6: Schwenkachse von 1
- 7: Zug-/Druckstange
- 8: Bolzen
- 9: Bohrung/Ausnehmung
- 10: schwenkbewegliches Griffteil
- 11: Grundkörper des Bedienteils
- 12: Gegengriffteil
- 13: Schwenkachse
- 14: Schraubenfeder
- 15: Ring
- 16: Nut
- 17: Abgeflachte Seiten des Bolzens
- 18: Anschlag
- 19: Ausnehmung

## Patentansprüche

1. Medizinisches Instrument, insbesondere endoskopisches Instrument, mit einem distalen Zangenmaulteil (1), mit einem proximalen Bedienteil (2) und mit einem diese verbindenden einseitig offenen Führungsteil (3), in dem eine Zug-/Druckstange (7) axial verschiebbar geführt ist, deren distales Ende an einem bewegbaren Teil (5) des Zangenmaulteils (1) oder einem damit verbundenen Bauteil angelenkt ist und deren proximales Ende in einer Arbeitsposition mit einem beweglich am Bedienteil (2) angeordneten Griffteil (10) bewegungsgekoppelt ist, wobei die Zug-/Druckstange (7) aus ihrer Arbeitsposition in eine Reinigungsposition verbringbar ist, in welcher zumindest ein Stangenabschnitt oder die gesamte Stange (7) außerhalb des Führungsteils (3) und das proximale Stangenende beabstanded zum Bedienteil (2) angeordnet ist und eine Spreizvorrichtung (8, 9, 14) vorgesehen ist, welche die Zug-/Druckstange (7) in ihrer Reinigungsposition hält, **dadurch gekennzeichnet, dass** die Spreizvorrichtung (8, 9, 14) zum selbsttätigen Überführen und Halten der Zug-/Druckstange (7) in die beziehungsweise in der Reinigungsposition ausgebildet ist und dass die Spreizvorrichtung (8, 9, 14) eine vorzugsweise vorgespannte Feder (14) aufweist, welche die Zug-/Druckstange (7) in Richtung von der Arbeitsposition zur Reinigungsposition hin kraftbeaufschlagt.

2. Medizinisches Instrument nach Anspruch 1, **dadurch gekennzeichnet, dass** das Führungsteil (3) durch einen einseitig zumindest abschnittsweise offenen Schaft gebildet ist.

3. Medizinisches Instrument nach einem der vorhergehenden Ansprüche, **dadurch gekennzeichnet, dass** eine Verriegelung (15, 16) vorgesehen ist, welche die Zug-/Druckstange (7) in ihrer Arbeitsposition festlegt.

4. Medizinisches Instrument nach Anspruch 3, **dadurch gekennzeichnet, dass** die Verriegelung einen das einseitig offene Führungsteil (3) abschnittsweise umgebenden Ring (15) aufweist, welcher drehbar am Führungsteil (3) und/oder am Bedienteil (2) vorzugsweise rastend festgelegt ist, und der eine Durchgangsnut (16) aufweist, welche in einer Drehstellung, in der die Durchgangsnut (16) mit der offenen Seite der Führung (3) fluchtet, einen Freiraum zum Hindurchführen der Zug-/Druckstange (7) bildet.

5. Medizinisches Instrument nach einem der vorhergehenden Ansprüche, **dadurch gekennzeichnet, dass** das Bedienteil (2) einen Grundkörper (11) und einen schwenkbar daran angeordneten Griffteil (10) aufweist, wobei im Griffteil (10) ein Bolzen (8) angeordnet ist, der mit einem Ende an der Zug-/Druckstange (7) angelenkt ist und mit seinem anderen Ende in einer Ausnehmung (9) im Griffteil (10) liegt, in welcher der Bolzen (8) axial beweglich geführt ist.

6. Medizinisches Instrument nach Anspruch 5, **dadurch gekennzeichnet, dass** die Feder (14) eine Schraubenfeder ist, welche in der Ausnehmung (9) des Griffteils (10) angeordnet ist und welche sich einerseits am Ende der Ausnehmung (9) im Griffteil (10) und andererseits am Bolzen (8) abstützt.

7. Medizinisches Instrument nach einem der vorhergehenden Ansprüche, **dadurch gekennzeichnet, dass** ein Anschlag (18) vorgesehen ist, welcher die Ausfahrbewegung des Bolzens (8) aus der Ausnehmung (9) begrenzt.

8. Medizinisches Instrument nach einem der vorhergehenden Ansprüche, **dadurch gekennzeichnet, dass** zwischen Bolzen (8) und Grundkörper (11) Formschlussmittel (17) vorgesehen sind, welche ein Ausfahren des Bolzens (8) nur bei einer vorgegebenen Stellung des Griffteils (10), vorzugsweise bei geöffneter Stellung des Griffteils (10), erlauben und welche die Schwenkbarkeit des Griffteils (10) in ausgefahrener Stellung des Bolzens (8) blockieren.

## Claims

1. A medical instrument, more particularly endoscopic instrument, with a distal jaw element (1), with a proximal operating element (2) and, connecting these, a guide element (3) which is open on one side and in which a pull/push rod (7) is guided in an axially displaceable manner, the distal end of which is articulated on a moveable element (5) of the jaw (1) or a component connected thereto, and the proximal end of which in an operating position is movably connected to a grip part (10) arranged on the operating element (2), wherein the pull/push rod (7) can be brought from its operating position into a cleaning position in which at least one rod section or the entire rod (7) is arranged outside the guide element (3) and the proximal rod end is arranged at a distance from the operating element (2) and a spreading device (8, 9, 14) is provided which holds the pull/push rod (7) in its cleaning position, **characterised in that** the spreading device (8, 9, 14) is designed for autonomously transferring and holding the pull/push device (7) into or in the cleaning position, and **in that** the spreading device (8, 9, 14) comprises a preferably pre-tensioned spring (14) which applies a force on the pull/push rod (7) in the direction from the operating position towards the cleaning position.

2. The medical instrument according to claim 1, **characterised in that** the guide element (3) is formed by a shaft that is at least in sections open on one side.

3. The medical instrument according to any one of the preceding claims, **characterised in that** a lock (15, 16) is provided which fixes the pull/push rod (7) in its operating position.

4. The medical instrument according to claim 3, **characterised in that** the lock has a ring (15) which surrounds the guide element (3) open on one side in sections and which is fastened rotatably on the guide element (3) and/or operating element (2), preferably in an engaging manner, and has a through groove (16), which in a rotary position in which the through groove (16) is flush with the open side of the guide element (3) creates a free space for passing through the pull/push rod (7).

5. The medical instrument according to any one of the preceding claims, **characterised in that** the operating element (2) comprises a basic body (11) and a grip part (10) pivotably arranged thereon, wherein arranged in the grip part (10) is a bolt (8), which is hinged with one end on the pull/push rod (7) and with its other end lies in a recess (9) in the grip part (10), in which the bolt (8) is axially moveable.

6. The medical instrument according to claim 5, **characterised in that** the spring (14) is a screw spring, which is arranged in the recess (9) of the grip part (10) and which on the one hand is supported on the end of the recess (9) in the grip part (10) and on the other hand on the bolt (8).

7. The medical instrument according to any one of the preceding claims, **characterised in that** a stop (18) is provided, which limits the extending movement of the bolt (8) out of the recess (9).

8. The medical instrument according to any one of the preceding claims, **characterised in that** between the bolt (8) and the basic body (11), positive locking means (17) are provided which only allow extension of the bolt (8) in the case of a predetermined position of the grip part (10), preferably in the open position of the grip part (10) and which block the pivotability of the grip part (10) when the bolt (8) is in the extended position.

## Revendications

1. Instrument médical, en particulier instrument endoscopique, avec une partie de mâchoire de pince distale (1), avec une partie de commande proximale (2) et avec une partie de guidage (3) qui les relie et qui est ouverte d'un côté et dans laquelle une tige de traction/pression (7) est guidée de manière à être mobile axialement, dont l'extrémité distale est articulée à une partie mobile (5) de la partie de mâchoire de pince (1) ou d'un composant qui est relié à celle-ci et dont l'extrémité proximale est couplée en déplacement dans une position de travail avec une partie de poignée (10) agencée de manière mobile sur la partie de commande (2), la tige de traction/pression (7) pouvant être déplacée de sa position de travail jusque dans une position de nettoyage dans laquelle au moins une section de tige ou la tige entière (7) est agencée à l'extérieur de la partie de guidage (3) et l'extrémité de tige proximale est agencée à distance de la partie de commande (2) et un dispositif d'écartement (8, 9, 14) est prévu, qui maintient la tige de traction/pression (7) dans sa position de nettoyage, **caractérisé en ce que** le dispositif d'écartement (8, 9, 14) est conçu respectivement pour le transfert et le maintien automatiques de la tige de traction/pression (7) dans la position de nettoyage, et **en ce que** le dispositif d'écartement (8, 9, 14) comprend un ressort (14) de préférence précontraint, qui maintient la tige de traction/pression (7) dans la direction de la position de travail vers la position de nettoyage sous l'action d'une force.

2. Instrument médical selon la revendication 1, **caractérisé en ce que** la pièce de guidage (3) est formée par une tige ouverte d'un côté au moins par sections.

3. Instrument médical selon l'une quelconque des revendications précédentes, **caractérisé en ce qu'**un verrouillage (15, 16) est prévu, qui fixe la tige de traction/pression (7) dans sa position de travail.

4. Instrument médical selon la revendication 3, **caractérisé en ce que** le verrouillage comprend une bague (15) entourant par sections la partie de guidage (3) ouverte d'un côté, qui est fixée de préférence par encliquetage de manière à pouvoir tourner sur la partie de guidage (3) et/ou sur la partie de commande (2), et qui comprend une rainure de passage (16) qui, dans une position de rotation dans laquelle la rainure de passage (16) est alignée avec le côté ouvert de la partie de guidage (3), forme un espace libre pour le passage de la tige de traction/pression (7).

5. Instrument médical selon l'une quelconque des revendications précédentes, **caractérisé en ce que** la partie de commande (2) comprend un corps de base (11) et une partie de poignée (10) agencée de manière pivotante sur celui-ci, un boulon (8) étant agencé dans la partie de poignée (10), qui est articulé par une extrémité sur la tige de traction/pression (7) et se trouve par son autre extrémité dans un évidement (9) dans la partie de poignée (10), dans lequel le boulon (8) est guidé de manière mobile axialement.

6. Instrument médical selon la revendication 5, **caractérisé en ce que** le ressort (14) est un ressort hélicoïdal qui est agencé dans l'évidement (9) de la partie de poignée (10) et qui prend appui d'une part à l'extrémité de l'évidement (9) dans la partie de poignée (10) et d'autre part sur le boulon (8).

7. Instrument médical selon l'une quelconque des revendications précédentes, **caractérisé en ce qu'**une butée (18) est prévue, qui limite le mouvement de sortie du boulon (8) à partir de l'évidement (9).

8. Instrument médical selon l'une quelconque des revendications précédentes, **caractérisé en ce qu'**il est prévu, entre le boulon (8) et le corps de base (11), des moyens de coopération de formes (17) qui ne permettent l'extension du boulon (8) que dans une position prédéterminée de la partie de poignée (10), de préférence dans la position ouverte de la partie de poignée (10), et qui bloquent la possibilité de pivotement de la partie de poignée (10) dans la position déployée du boulon (8).
